# EUROPEAN PATENT SPECIFICATION

(11) **EP 1 290 992 B1**
(45) Date of publication and mention of the grant of the patent: **30.08.2006**
(21) Application number: 02256257.3
(22) Date of filing: 10.09.2002
(51) Int. Cl.: A61F 2/34, A61L 27/10, A61F 2/32

(54) **Acetabular cup**
Hüftgelenkspfanne
Cupule cotyloidienne

(30) Priority: 11.09.2001 GB 0122002
(43) Date of publication of application: 12.03.2003
(73) Proprietor: BENOIST GIRARD SAS, 14201 Hérouville-Saint-Clair Cédex (FR)
(72) Inventor: Cueille, Christophe, 14210 Missy (FR); Pichon, Denis, 35310 Mordelles (FR)
(74) Representative: Bridge-Butler, Alan James

(56) References cited:
- WO-A-99/30634
- GB-A- 1 334 584
- US-A- 4 179 485
- US-A- 4 801 300
- US-A- 5 658 348
- US-A- 5 716 414
- US-A- 5 980 574

## Description

This invention relates to a method of manufacturing an acetabular cup for a total hip prosthesis and a total hip prosthesis embodying such a cup.

A total replacement hip joint comprises of two general prosthetic components which are implanted into the patient's hip and leg. A femoral ball component replaces the natural femoral head surface, and an acetabular cup component replaces the natural acetabular socket surface.

Femoral prosthetic components come in many different forms in order to suit the patient and to minimise the amount of natural bone which has to be removed. Some of these components are approximately hemispherical in shape and are fitted directly onto the femoral head. Other components comprise a stem portion which is implanted into the medullary canal, and a neck carrying a ball head, or a head spigot adapted to carry a ball head.

Acetabular cup components are also made in a variety of forms in order to suit the patient's needs. These components are generally hemispherical in shape and are fitted into the acetabulum.

It is important in a total replacement hip joint for the femoral head and the acetabular cup to have good bearing surfaces in order to provide a natural smooth hip movement. It is known to construct hip replacement components from metals such as titanium or stainless steel alloys, plastics materials such as polyethylene, and ceramics materials such as alumina.

However, the materials used must also be able to withstand loading of the joint post surgery. During natural gait and other natural movements various multi-directional loads are applied to the joint. In order to withstand these forces an acetabular cup must be either very strong, or attached uniformly to the acetabulum so the loads are dispersed across its spherical structure. It is known for prostheses to fail if they are not attached correctly to the bone because the loads become concentrated in small areas.

The most desired form of total hip prosthesis is a ceramics on ceramics arrangement with both the femoral ball head and the acetabular cup being constructed from a ceramics material. Further, it has been found that the bearing surface with a ceramics on ceramics arrangement works best with the known larger type of prosthetic ball heads and sockets which are of similar dimensions to the natural hip joint. These larger scale prosthetic joints also provide more stability during natural gait, and reduces the possibility of dislocation and the present invention is for use in this type of larger scale prosthetic joint.

However, such an arrangement has not been thought possible because of the known difficulty in attaching a ceramic acetabular cup to the acetabulum. Ceramics materials are relatively brittle, and are known to fail without a uniform interface with the bone to distribute the load across the bone. Attempts have been made to attach larger size ceramic acetabular cups to the bone with cement and other known means, but they did not achieve a satisfactory attachment, which led to eventual failure when the load became concentrated in small areas. So far only ceramics acetabular cups provided with an outer metallic bone-interfacing shell have been successful. However, the outer layer increases the wall thickness of the cup, which reduces the interior space available for the bearing surface of the joint. Therefore these acetabular cups do not benefit from the superior bearing surface and stability which is provided by the known larger type of prosthetic ball head and socket arrangements described above.

WO 99/30634A shows a hip socket in which the bearing shell of the socket that mounts the ball head of the shaft is made of a ceramic material and which in the region of its surface with which it is inserted into the hip bone is provided with a coating that is made of a biocompatible metal or a biocompatible metal alloy which can be provided with a bioactive covering. In this construction the coating of biocompatible metal or metal alloy is vapour deposited or sprayed onto the ceramic bearing shell. There are difficulties with this type of construction in as much that it is difficult to provide a good attachment between the coating and the bearing shell.

US 5,658,348 also shows a construction in which an acetabular cup can be provided with a ceramic liner with an outer shell made from a biocompatible material such as cobalt-chromium or titanium alloys. In this construction the outer shell is connected to the liner by fixation screws and includes a threaded polar protrusion having threads which mate with threads in a polar hole in the shell. Again, this type of construction is not sufficiently positive for use with larger scale prosthetic joints.

It is therefore an object of the present invention to provide a method of manufacturing an acetabular cup which provides means to successfully attach a large-sized ceramic acetabular cup to the acetabulum for use with a large-sized ceramic femoral ball head.

According to the present invention a method of manufacturing an acetabular cup for use in a total hip prosthesis employing a large sized femoral ball head which is of similar dimensions to a natural hip joint which comprises a hemispherical inner bearing surface portion constructed from a ceramics material, a hemispherical outer bone-interface layer constructed from titanium or a titanium alloy, and an outer coating of a bone growth stimulant substance applied to the outer bone-interface layer, characterised by including the steps of:
Forming the bone-interface layer as a preformed hemispherical support shell.
Heating the support shell.
Applying the support shell to the inner bearing surface portion.
Allowing the support shell to shrink and form a secure fitting on the inner bearing surface portion.

A preferred method may include applying a compressive force between the support shell (3) and the inner bearing surface portion (2) during shrinkage.

The inner bearing surface portion can be constructed from alumina, or zirconia toughened alumina (ZTA). The bone growth stimulant can be any suitable compound. In a preferred embodiment a hydroxyapatite coating is used. This material is known to be a successful bone-growth stimulant. Its presence on the hemispherical support shell stimulates the bone to grow around or into the layer, holding it firmly in place.

The present invention can be performed in various ways and some methods will now be described by way of example and with reference to the accompanying drawings in which:
Figure 1 is a cross-sectional side view of an acetabular cup made by the method according to the present invention; and,
Figure 2 is a cross-sectional side view of a total prosthetic hip joint incorporating an acetabular cup made by the method and implanted according to the present invention.

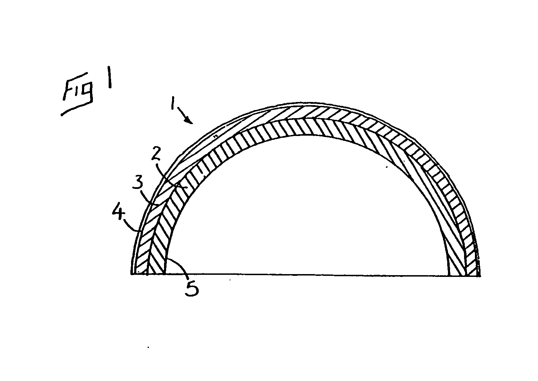

In Figure 1 an acetabular cup according to the present invention 1 comprises an inner bearing surface portion 2, an outer bone-interface layer 3 and a hydroxyapatite coating 4. The inner bearing surface portion 2 has a bearing surface 5, and is constructed from a ceramics material, for example dense ZTA. The outer bone-interface layer 3 is a titanium alloy support shell, for example made from TA6V, which has been attached to the inner bearing surface portion by shrinkage after heating during manufacture. Thus, according to the invention, the method includes the steps of:
Forming the bone interface layer as a preformed hemispherical support shell.
Heating the support shell.
Applying the support shell to the inner bearing surface portion.
Allowing the support shell to shrink and form a secure fitting on the inner bearing surface portion.

A compressive force can be applied between the support shell and the inner bearing surface portion during shrinkage.

With this method the surface finish of the bone interface layer can be structured, by machining or any other method, before the HA coating is applied.

In the acetabular cup in Figure 1 the inner bearing surface portion can be between 3-5 millimetres thick, the outer bone-interface layer can be between 0.1 - 2 millimetres thick and the hydroxyapatite coating can be between 0.020 to 0.2 millimetres thick.

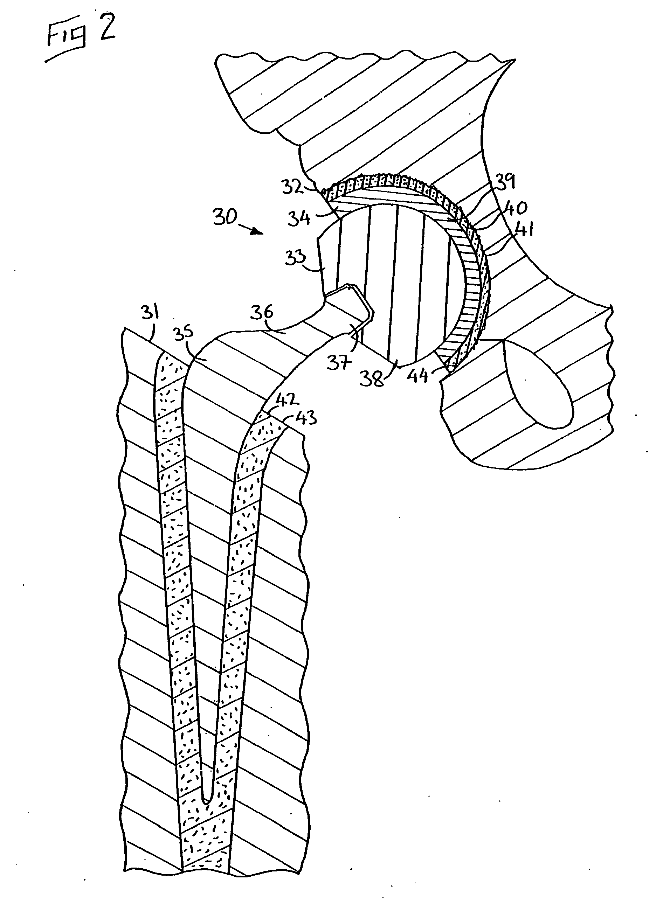

In Figure 2 a total replacement hip joint using a cup made to the method set forth above and according to the present invention 30 has been implanted into the femur 31 and the acetabulum 32. The hip joint 30 comprises a femoral stem component 33 and an acetabular cup component 34. The femoral stem component 33 comprises a body 35, a neck 36, a spigot 37 and a large ball head 38 made from alumina and mounted on said spigot 37. The acetabular cup component 34 comprises an inner bearing surface portion 39 constructed from a ceramics material, an outer bone-interface layer 40 made from a titanium alloy, and a hydroxyapatite coating 41.

As shown in Figure 2 the femoral stem component 33 has been successfully implanted into the medullary canal 42 with the use of cement 43. The acetabular cup 34 has been successfully implanted into the acetabulum 32. The exposed bony matter of the acetabulum 44 has interfaced with the outer bone-interface layer 40 thanks to the hydroxyapatite bone growth stimulant 41. With this arrangement the multi-directional loading of the prosthetic hip joint will not result in prosthesis failure because the load will be carried across the sphere of the acetabular cup. Further, the ceramics on ceramics bearing provides smooth movement, and the large size of the joint provides stability during movement and increased security from dislocation.

Therefore, a large-sized ceramic acetabular cup is provided which can be successfully attached to the acetabulum for use with a large sized ceramic femoral head component.

Further, a total hip replacement joint is provided with the benefits of a natural-sized ceramics on ceramics bearing surface.

## Claims

1. A method of manufacturing an acetabular cup for use in a total hip prosthesis employing a large sized femoral ball head (38) which is of similar dimensions to a natural hip joint which comprises a hemispherical inner bearing surface portion (7) constructed from a ceramics material, a hemispherical outer bone-interface layer (3) constructed from titanium or a titanium alloy, and an outer coating (4) of a bone growth stimulant substance applied to the outer bone-interface layer (3), **characterised by** including the steps of:
Forming the bone-interface layer as a preformed hemispherical support shell (3).
Heating the support shell (3).
Applying the support shell (3) to the inner bearing surface portion (2).
Allowing the support shell (3) to shrink and form a secure fitting on the inner bearing surface portion (2).

2. A method of manufacturing an acetabular cup as claimed in Claim9 **characterised by** applying a compressive force between the support shell (3) and the inner bearing surface portion (2) during shrinkage

3. A method of manufacturing an acetabular cup as claimed in Claim 1 or Claim 2 **characterised in that** the inner bearing surface portion (2) is constructed from alumina.

4. A method of manufacturing an acetabular cup as claimed in Claim 1 or Claim 2 **characterised in that** the inner bearing surface portion (2) is constructed from zirconia toughened alumina.

5. A method of manufacturing an acetabular cup as claimed in any of the above Claims 1 to 4 **characterised in that** the bone growth stimulant substance is a hydroxyapatite coating.

6. A method of manufacturing an acetabular cup as claimed in any of the above Claims 1 to 5 **characterised in that** the inner bearing surface portion (2) is between 3 and 5 millimetres thick.

7. A method of manufacturing an acetabular cup as claimed in any of the above Claims 1 to 6 **characterised in that** the hemispherical support shell (3) is between 0.1 and 2 millimetres thick.

8. A method of manufacturing an acetabular cup as claimed in any of the above Claims in which the outer coating is between 0.020 and 0.2 millimetres thick.

## Patentansprüche

1. Verfahren zur Herstellung einer Hüftpfanne zur Verwendung in einer totalen Hüftprothese, die einen groß bemessenen femoralen Kugelkopf (38) verwendet, der ähnliche Abmessungen wie ein natürliches Hüftgelenk aufweist, welche einen aus einem Keramikmaterial konstruierten halbkugelförmigen inneren Lageroberflächenteil (7), eine aus Titan oder einer Titanlegierung konstruierte halbkugelförmige äußere Knochengrenzflächenschicht (3), sowie eine auf die äußere Knochengrenzflächenschicht (3) aufgebrachte äußere Beschichtung (4) aus einer Knochenwachstum stimulierenden Substanz umfasst, **dadurch gekennzeichnet, dass** es die Schritte einschließt:
Bilden der Knochengrenzflächenschicht als vorgeformte halbkugelförmige Auflageschale (3).
Erwärmen der Auflageschale (3).
Anlegen der Auflageschale (3) am inneren Lageroberflächenteil (2).
Zulassen, dass die Auflageschale (3) schrumpft und einen sicheren Sitz auf dem inneren Lageroberflächenteil (2) bildet.

2. Verfahren zur Herstellung einer Hüftpfanne nach Anspruch 1, **gekennzeichnet durch** Aufbringen einer Druckkraft zwischen der Auflageschale (3) und dem inneren Lageroberflächenteil (2) während des Aufschrumpfens.

3. Verfahren zur Herstellung einer Hüftpfanne nach Anspruch 1 oder Anspruch 2, **dadurch gekennzeichnet, dass** der innere Lageroberflächenteil (2) aus Aluminiumoxid konstruiert wird.

4. Verfahren zur Herstellung einer Hüftpfanne nach Anspruch 1 oder Anspruch 2, **dadurch gekennzeichnet, dass** der innere Lageroberflächenteil (2) aus mit Zirkoniumoxid verstärktem Aluminiumoxid konstruiert wird.

5. Verfahren zur Herstellung einer Hüftpfanne nach einem der obigen Ansprüche 1 bis 4, **dadurch gekennzeichnet, dass** die Knochenwachstum stimulierende Substanz eine Hydroxylapatit-Beschichtung ist.

6. Verfahren zur Herstellung einer Hüftpfanne nach einem der obigen Ansprüche 1 bis 5, **dadurch gekennzeichnet, dass** der innere Lageroberflächenteil (2) zwischen 3 und 5 Millimeter dick ist.

7. Verfahren zur Herstellung einer Hüftpfanne nach einem der obigen Ansprüche 1 bis 6, **dadurch gekennzeichnet, dass** die halbkugelförmige Auflageschale (3) zwischen 0,1 und 2 Millimeter dick ist.

8. Verfahren zur Herstellung einer Hüftpfanne nach einem der obigen Ansprüche, bei dem die äußere Beschichtung zwischen 0,020 und 0,2 Millimeter dick ist.

## Revendications

1. Procédé pour fabriquer une coupelle cotyloïdienne destinée à être utilisée dans une prothèse totale de la hanche employant une tête à bille fémorale de grande taille (38) qui est de dimensions similaires à une articulation naturelle de la hanche, qui comprend une partie de surface de portée intérieure hémisphérique (7) construite en un matériau céramique, une couche d'interface avec l'os extérieure hémisphérique (3) construite en titane ou en un alliage de titane, et un revêtement extérieur (4) en une substance stimulant la croissance osseuse appliquée à la couche d'interface avec l'os extérieure (3), **caractérisé en ce qu'**il comprend les étapes consistant à :
former la couche d'interface avec l'os sous la forme d'une coque de support hémisphérique préformée (3),
chauffer la coque de support (3),
appliquer la coque de support (3) à la partie de surface de portée intérieure (2),
laisser la coque de support (3) se rétracter et former une fixation ferme sur la partie de surface de portée intérieure (2).

2. Procédé pour fabriquer une coupelle cotyloïdienne selon la revendication 1, **caractérisé par** l'application d'une force de compression entre la coque de support (3) et la partie de surface de portée intérieure (2) durant la rétraction.

3. Procédé pour fabriquer une coupelle cotyloïdienne selon la revendication 1 ou la revendication 2, **caractérisé en ce que** la partie de surface de portée intérieure (2) est construite en alumine.

4. Procédé pour fabriquer une coupelle cotyloïdienne selon la revendication 1 ou la revendication 2,
**caractérisé en ce que** la partie de surface de portée intérieure (2) est construite en alumine renforcée à la zircone.

5. Procédé pour fabriquer une coupelle cotyloïdienne selon l'une quelconque des revendications 1 à 4 ci-dessus, **caractérisé en ce que** la substance stimulant la croissance osseuse est un revêtement d'hydroxyapatite.

6. Procédé pour fabriquer une coupelle cotyloïdienne selon l'une quelconque des revendications 1 à 5 ci-dessus, **caractérisé en ce que** la partie de surface de portée intérieure (2) a une épaisseur comprise entre 3 et 5 millimètres.

7. Procédé pour fabriquer une coupelle cotyloïdienne selon l'une quelconque des revendications 1 à 6 ci-dessus, **caractérisé en ce que** la coque de support hémisphérique (3) a une épaisseur comprise entre 0,1 et 2 millimètres.

8. Procédé pour fabriquer une coupelle acétabulaire selon l'une quelconque des revendications ci-dessus, dans lequel le revêtement extérieur a une épaisseur comprise entre 0,020 et 0,2 millimètre.
